# FASCICULE DE BREVET EUROPEEN

(11) **EP 3 270 789 B1**
(45) Date de publication et mention de la délivrance du brevet: **26.12.2018**
(21) Numéro de dépôt: 16706644.8
(22) Date de dépôt: 26.02.2016
(51) Int. Cl.: A61B 6/14, A61B 6/00

(54) **SYSTEME D'AIDE AU POSITIONNEMENT D'UN CAPTEUR RADIOLOGIQUE DENTAIRE INTRA-ORAL**
SYSTEM ZUR UNTERSTÜTZUNG DER POSITIONIERUNG EINES INTRAORALEN DENTALEN RADIOLOGIESENSORS
SYSTEM FOR ASSISTING IN THE POSITIONING OF A INTRAORAL DENTAL RADIOLOGY SENSOR

(30) Priorité: 20.03.2015 FR 1552344
(43) Date de publication de la demande: 24.01.2018
(73) Titulaire: Teledyne E2V Semiconductors SAS, 38120 Saint-Egrève (FR)
(72) Inventeur: PASCAL, Nathalie, 38700 Le Sappey En Chartreuse (FR); JULIEN, Florian, 38000 Grenoble (FR)
(74) Mandataire: Desvignes, Agnès
(86) Numéro de dépôt international: PCT/EP2016/054142
(87) Numéro de publication internationale: WO 2016/150654

(56) Documents cités:
- US-A- 4 012 638
- US-A1- 2006 274 890
- US-B1- 8 308 360

## Description

L'invention concerne l'imagerie médicale et plus particulièrement la radiologie dentaire intra-orale. Les systèmes radiologiques dentaires utilisent un capteur d'image qui est placé dans la bouche du patient, derrière et à proximité de la région dentaire à observer. Une source de rayons X est placée à l'extérieur de la bouche du patient, en face du capteur et expose celui-ci à un court flash de rayons X, à travers les tissus biologiques ou autres matières à observer qui sont situés entre la source et la face active du capteur. La face active du capteur est la face qui est sensible aux radiations, et qui permet d'obtenir le cliché radio de la zone anatomique que l'on souhaite observer.

Parmi les contraintes importantes d'utilisation des systèmes radiologiques dentaires, il faut considérer tout particulièrement le risque d'exposition du patient et de son entourage aux rayons X. Il est nécessaire de minimiser la dose de rayons X envoyée, tout en faisant en sorte d'obtenir une bonne image de la région observée.

Obtenir la bonne image suppose que le capteur soit placé correctement dans la bouche du patient, relativement à la zone anatomique que l'on souhaite radiographier, faute de quoi une partie de la dose envoyée ne servirait à rien.

Mais placer correctement le capteur dans la bouche d'un patient est une opération qui peut se révéler difficile dans la pratique, à cause de l'obscurité dans la cavité buccale, de son volume limité et de l'accès plus ou moins aisé (diamètre d'ouverture de la bouche variable selon les patients).

Pour toutes ces raisons, le praticien peut avoir des difficultés à positionner correctement la face active du capteur par rapport à la partie anatomique du patient qu'il souhaite radiographier (dents, racines ...) et à vérifier visuellement, de manière directe, et corriger si nécessaire, ce positionnement. La vérification se fera donc de manière indirecte, sur le cliché obtenu : si le positionnement n'est pas bon, le cliché obtenu ne sera d'aucune utilité au praticien, qui devra prendre un nouveau cliché radiographique. Le document US4012638 divulgue un ensemble de prise d'image comprenant un capteur d'image intra oral avec des réflecteurs infrarouge.

Or on cherche à minimiser l'exposition des personnes aux rayonnements autant que raisonnablement possible.

Des accessoires supports sont proposés aux praticiens pour faciliter la mise en place correcte du capteur d'image ainsi que le maintien dans la bonne position le temps de réaliser la radiographie.

Certains de ces accessoires supports sont spécialement conçus pour réaliser des clichés bien précis. Ce sont par exemple des accessoires comportant une zone support du capteur et une zone à mordre.

D'autres accessoires supports permettent tous les types de clichés. Ces accessoires comportent généralement une zone support de capteur au bout d'une tige de guidage et maintien qui facilite la manipulation par le praticien.

Ces accessoires supports facilitent le placement et la manipulation, certes, mais ils ne sont pas suffisants à assurer à chaque fois un bon positionnement du capteur, tout simplement parce que la bouche et la dentition de chaque patient sont uniques. A chaque fois il y a un réel ajustement à faire. Et le praticien a une grande difficulté à pouvoir réaliser et vérifier cet ajustement, à cause de l'obscurité de la cavité buccale.

### RESUME DE L'INVENTION

Un objet de l'invention est ainsi d'aider au positionnement du capteur dentaire intra-oral.

L'invention propose un ensemble de prise d'image radiologique comprenant un capteur d'image radiologique intra-oral, le capteur comprenant une face active et une face arrière, la face active étant destinée à être tournée vers une source de rayons X.

Selon l'invention, cet ensemble comprend au moins un miroir qui est placé devant la face active du capteur, le miroir formant une face réfléchissant la lumière visible qui est tournée du même côté que la face active du capteur.

Dans un mode de réalisation, un miroir est formé sur une face externe d'un boîtier d'encapsulation enfermant le capteur, où la face externe correspond à la face active du capteur. Le miroir peut avoir été préalablement formé sur le capot de face avant du boitier, avant encapsulation et soudage du capot avec une coque de face arrière du boitier.

Selon l'invention, l'ensemble de prise d'image peut comprendre au moins un accessoire supportant ou contenant le capteur où une face externe de l'accessoire est placée devant la face active du capteur et tournée dans le même sens que la face active du capteur, et un miroir formé sur cette face externe de l'accessoire.

L'accessoire peut être un outil comprenant une zone support solidaire d'une tige, où la zone support est prévue pour tenir le capteur, face active du capteur contre face interne de la zone support. Le miroir peut alors être formé sur une face externe de la zone support.

L'accessoire peut être une enveloppe de protection hygiénique, jetable ou non, prévue pour enfermer un capteur intra-oral. Le miroir peut alors être formé sur la surface externe de l'enveloppe.

L'accessoire peut être une coque souple de confort à l'intérieur de laquelle le capteur est placé, face active contre une paroi de fond de la coque et un miroir est formé sur la face extérieure de cette paroi.

L'ensemble de prise d'image peut comprendre un ou plusieurs de ces accessoires, et au moins la face (la plus) externe de l'ensemble de prise d'image, qui est celle d'un des accessoires, comprend un miroir selon l'invention.

Le miroir peut être formé par dépôt en couche d'un matériau réfléchissant, par évaporation sous vide, sur un capot de face avant de boitier avant encapsulation, et/ou sur la face avant du boitier d'encapsulation enfermant le capteur et/ou sur la face externe de la coque de confort et/ou de la zone support d'un accessoire.

Le miroir peut encore être formé par une pièce de miroir découpée dans une bande de matériau ayant une face réfléchissante et intégrée par moulage dans un matériau thermoplastique.

Le miroir peut encore être formé par découpe et collage d'une pièce de miroir dans une bande de matériau ayant une face réfléchissante et une autre face adhésive.

D'autres caractéristiques et avantages de l'invention apparaîtront à la lecture de la description détaillée qui suit et qui est faite en référence aux dessins annexés dans lesquels :
- la figure 1 illustre le principe d'une prise de vue radiologique dentaire ;
- la figure 2 est une vue de face avant d'un capteur radiologique dentaire intra-oral ;
- la figure 3 est une vue de face arrière d'un capteur radiologique dentaire intra-oral ;
- la figure 4 est une vue en coupe de ce capteur, montrant le montage de la puce électronique du capteur, encapsulée dans un boitier ;
- la figure 5 illustre un exemple de miroir d'aide au positionnement selon l'invention formé en face avant d'un capteur radiologique ;
- la figure 6 illustre un autre exemple de miroir d'aide au positionnement selon l'invention formé sur une face extérieure d'un accessoire support d'un capteur radiologique ;
- les figures 7 et 8 représentent un autre exemple de miroir d'aide au positionnement selon l'invention formé sur une face extérieure d'un accessoire de protection hygiénique d'un capteur radiologique, qui est une enveloppe dans laquelle on vient insérer et enfermer le capteur ;
- la figure 9 représente une coque de confort amovible de capteur radiologique ; et
- la figure 10 représente un autre exemple de miroir d'aide au positionnement selon l'invention formé sur une face extérieure d'une telle coque de confort ; et
- la figure 11 illustre une pièce prédécoupée en matériau réfléchissant, pour réaliser un miroir d'aide au positionnement selon l'invention.

### DESCRIPTION DETAILLEE DE L'INVENTION

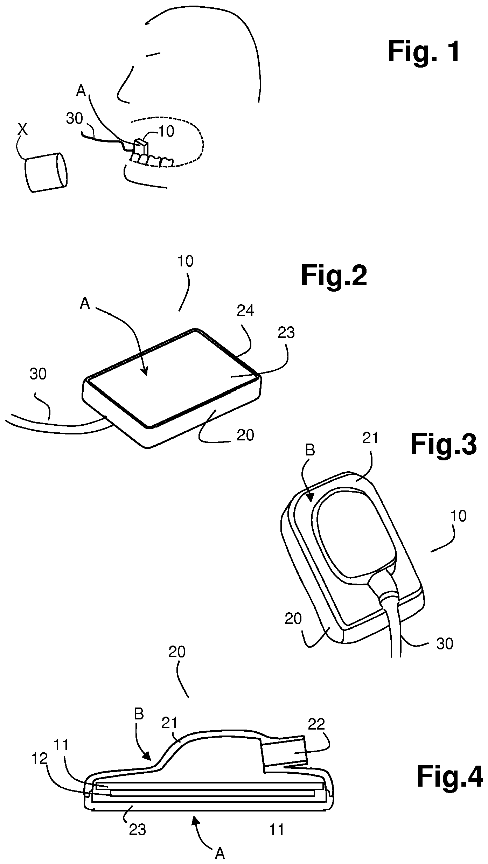

Sur la figure 1, on a représenté de manière simplifiée le principe et les éléments de prise d'image radiologique dentaire : un capteur radiologique dentaire intra-oral 10 est placé dans la bouche d'un patient, sa face active A derrière la zone anatomique à radiographier. Une source externe de rayons X est placée à l'extérieur de la bouche, en alignement avec la zone à radiographier et la face active A du capteur.

Dans l'exemple, le capteur comprend un câble de sortie 30 destiné à être relié à un ordinateur. Le câble va permettre la transmission d'image et d'autres signaux d'interface ou de commande éventuels et son alimentation électrique ou la recharge d'une batterie intégrée au capteur. Mais une interface sans fil est possible.

Il faut comprendre par capteur, l'ensemble comprenant un scintillateur, un circuit électronique qui réalise la capture d'image et la transmission de l'image, et un boitier de protection dans lequel ce circuit est encapsulé. Le circuit électronique peut être en toute technologie de l'état de l'art (CCD, MOS). Comme illustré sur la vue en coupe de la figure 4, l'ensemble comprenant le circuit électronique 11 et le scintillateur 12 est placé (éventuellement avec d'autres éléments non représentés telle que des éléments de rigidification, des composants discrets, une batterie...) dans une coque 21 dont le fond forme la face arrière B (figure 3) du boitier, avec une ouverture 22 prévue pour permettre le passage d'un câble électrique de sortie 30. Un capot de face avant 23 (figure 2) est rapporté par-dessus le circuit et assemblé à la coque, typiquement par soudage ultra-sons. Le capot de face avant 23 correspond à la face active A du capteur : sous ce capot 23 on va ainsi trouver le scintillateur 12, et sous le scintillateur, la matrice d'éléments photosensibles du circuit électronique 11. Les radiations X émises par une source placée devant cette face active, sont transformées par le scintillateur en photons lumineux détectables par les éléments photosensibles de la matrice.

La coque 21 et le capot de face avant 23 qui forment le boitier 20 sont conçus de manière à apporter la rigidité nécessaire pour protéger le circuit électronique et spécialement la matrice photosensible, à permettre une sortie étanche du câble de sortie 30 et à assurer également un confort en bouche pour le patient. Notamment : le boîtier, de forme rectangulaire peut présenter des coins coupés et des bords arrondis ; le capot de face avant peut être en matériau plastique dur ; les bords du boitier peuvent être en plastique souple à aspect de gomme lisse.

Pour aider le praticien à bien positionner le capteur dans la bouche d'un patient, l'invention propose de placer au moins un miroir devant la face active du capteur, avec la surface réfléchissante du miroir tournée dans le même sens que la face active A, c'est-à-dire vers la zone à radiographier et vers la source externe de rayons X (Figure 1).

Plus précisément, selon l'invention, un ensemble de prise d'image radiologique comprenant un capteur d'image intra-oral comprend un miroir disposé sur une face externe de l'ensemble de prise d'image, avec une face réfléchissante du miroir orientée du même côté que la face active A du capteur.

L'ensemble de prise d'image radiologique (ou une partie de celui-ci, incluant bien sûr le capteur d'image) est placé dans la bouche du patient, derrière la zone anatomique à radiographier : sur la surface de ce miroir, éclairée par la lumière visible (naturelle, ou plus efficacement la lumière émise par un appareil d'éclairage orienté vers la bouche du patient), va ainsi se former une image de la zone anatomique derrière laquelle le capteur est placé : le praticien va pouvoir s'aider de cette image, pour ajuster le positionnement du capteur relativement à la zone qu'il souhaite radiographier. Le miroir sera en pratique le plus transparent possible aux rayons X pour ne pas altérer la qualité de l'image radiologique.

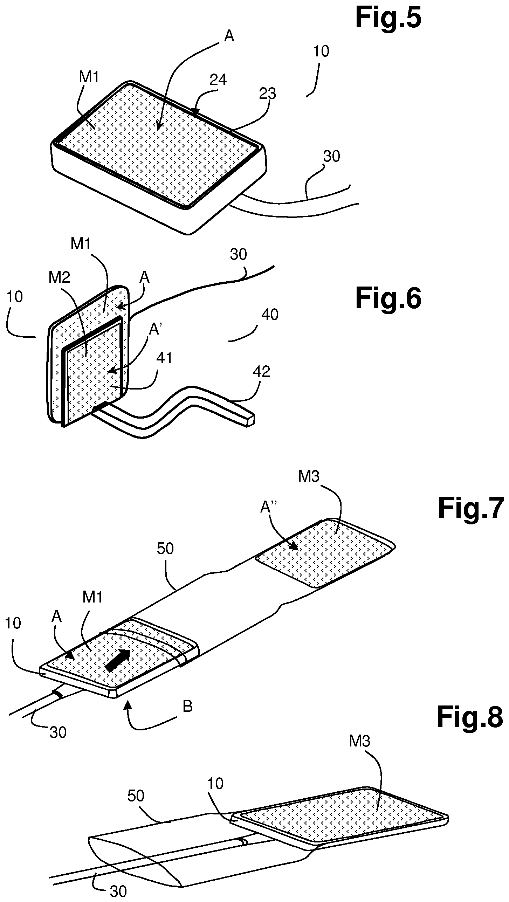

Dans une mise en oeuvre de l'invention, un miroir d'aide au positionnement selon l'invention est intégré au capteur lui-même, comme illustré sur la figure 5 : un miroir M1 est formé sur la face avant externe du boitier 20 d'encapsulation du capteur correspondant à la face active A du capteur : c'est-à-dire en reprenant l'exemple de boitier illustré sur les figures 2 à 4, sur le capot 23 de face avant de ce boitier, sur toute la surface de ce capot à l'exception de la bordure 24. Dans cette réalisation, la surface du miroir M1 est sensiblement égale et exactement positionnée, en superposition, dans l'axe de la source de rayons X à la surface de matrice photosensible du capteur : l'image visible qui se forme sur le miroir est proche de l'image radiographique qui sera obtenue, et le praticien peut réaliser le positionnement du capteur avec précision en observant cette image.

Le miroir M1 peut être formé avant ou après encapsulation du capteur dans son boîtier 20. S'il est réalisé après encapsulation, on pourra utiliser une technique de dépôt d'un matériau réfléchissant par évaporation en phase vapeur, avec un outillage de masquage pour délimiter la zone de dépôt et déposer le matériau réfléchissant partout sur la face interne du capot 23, à l'exception de la bordure 24.

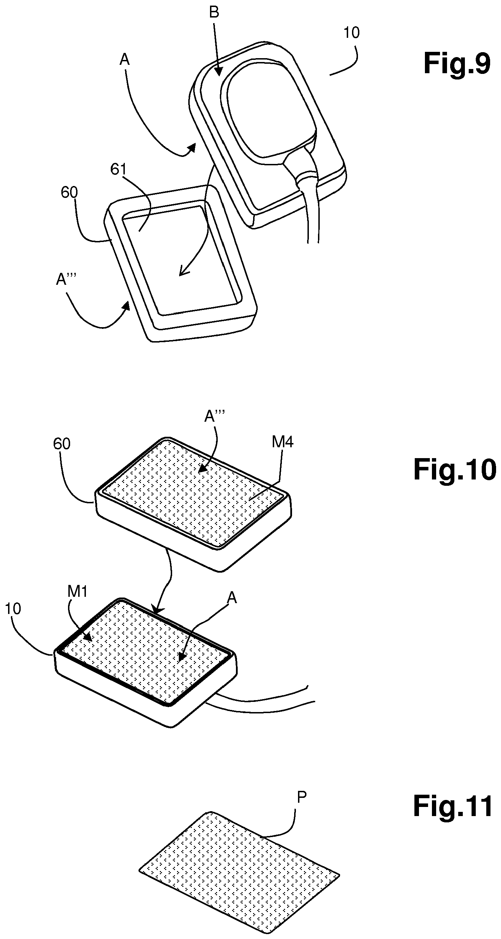

On peut aussi réaliser le miroir avant encapsulation. Plus précisément, on peut prévoir de réaliser le miroir M1 au moment de la fabrication du capot 23 de face avant, avant encapsulation du circuit et assemblage à la coque 21 de face arrière. Dans cette variante on peut utiliser la même technique de dépôt par évaporation. Mais on pourrait aussi prédécouper une pièce P de miroir (figure 11), dans une bande de matériau ayant une face réfléchissante, et plaquer cette pièce P au fond d'un moule pour mouler par-dessus et autour de cette pièce, la forme de capot de face avant. La pièce de miroir est alors intégrée dans le capot, en surface de ce capot. On pourrait utiliser la pièce P de miroir comme fond du capot et former la bordure 24 par surmoulage de matériau thermoplastique.

On peut encore prévoir de rapporter la pièce P de miroir sur le capot de face avant du boitier par collage. De préférence on peut alors prévoir de fournir la pièce P de miroir avec une face adhésive, utilisant un adhésif sensible à la pression, qui est protégée par un film pelable. Le miroir peut alors être mis en place par le praticien sur le capteur, c'est-à-dire sur la face avant du boitier d'encapsulation.

Les ensembles de prise d'image utilisés par les praticiens peuvent aussi comprendre un ou des accessoires en plus du capteur. Il y a les accessoires prévus pour aider à la manipulation et au placement du capteur dans la bouche du patient, les accessoires d'hygiène, ou encore les accessoires prévus pour améliorer le confort des patients : quel que soit l'accessoire ou les accessoires utilisés, on formera de préférence un miroir sur la surface externe de l'ensemble de prise d'image, c'est-à-dire celle qui vient au premier plan, vue du côté du praticien qui regarde vers l'intérieur de la bouche du patient, que ce soit une face du boitier du capteur ou celle d'un accessoire qui se place dans la bouche entre la zone à radiographier et la face active du capteur.

Ces accessoires sont réalisés dans des matériaux adaptés à l'usage médical ou dentaire (à usage unique ou non) et transparents aux radiations X, et notamment des matériaux plastiques, qui ne sont en général pas transparents à la lumière visible, et qui pour cette raison, peuvent masquer ou rendre moins visible (pour le praticien), au moins en partie, la face active du capteur.

Un premier accessoire peut être un élément support 40 (*"handler"*) de capteur comme illustré sur la figure 6. L'élément support de capteur comprend un support 41 de capteur, prévu pour y placer et tenir un capteur 10, et une tige 42 solidaire de la zone support 41, que le praticien va pouvoir manipuler. La tige peut ou non être articulée. Elle peut aussi être couplée à d'autres éléments (non représentés), tel que par exemple une fenêtre d'alignement de la source de rayons X, ou un élément à mordre.

Le support 41 comporte une face externe A', côté tige et une face interne prévue pour recevoir le capteur, face active A du capteur 10 contre cette face interne et pour maintenir le capteur dans cette position : à cet effet la face interne peut inclure des éléments mécaniques non représentés, tels que des glissières ou des pinces par exemple, ou bien cette face interne est une face adhésive, avec un adhésif du type sensible à la pression (PSA) ....

Le support 41 se présente, comme illustré sur la figure 6, comme une surface sensiblement plane qui masque en partie au moins la face active A du capteur. Dans l'exemple illustré, la surface est pleine. Mais elle pourrait comporter une ou des ouvertures.

Selon l'invention, un miroir M2 peut être prévu sur la face externe A' du support 41, face réfléchissante tournée du même côté que la face active A du capteur, c'est-à-dire vers la zone à radiographier. Si, comme représenté, le capteur comprend un miroir M1 sur sa face active A, les deux miroirs M1 et M2 sont orientés du même côté, c'est-à-dire pour faire face à la source externe de rayons X (Figure 1).

Le miroir M2 peut être réalisé sur la face A' du support 41 en utilisant les mêmes techniques indiquées supra : évaporation sous vide ; ou bien, lorsqu'au moins le support 41 est réalisé par moulage d'un matériau thermoplastique, intégration en surface de ce support par prédécoupe d'une pièce de miroir et moulage par-dessus et autour de cette pièce de la forme de support (ou d'élément support 40) ; ou encore, collage d'une pièce P de miroir.

Lorsque le support 41 est conformé pour correspondre aux dimensions exactes du capteur, par exemple dans le cas d'un accessoire sur-mesure, les dimensions du miroir M2 vont pouvoir avantageusement correspondre aux dimensions de la matrice photosensibles du capteur. Dans ce cas, le miroir M2 formé sur le support 41 permet le positionnement le plus précis du capteur, car l'image qui va se former sur ce miroir va correspondre au plus près à l'image radiologique que le capteur pourra détecter. Dans cette situation, le boitier du capteur n'a pas besoin d'être revêtu d'un miroir. Il en est de même si le support 41 est plus grand que le capteur.

Dans le cas où l'élément de support est générique, les dimensions du miroir réalisé sur la face externe A' du support de capteur vont dépendre en pratique de la surface de ce support. Dans tous les cas on cherchera à favoriser la précision du positionnement : c'est-à-dire que l'on cherchera à faire correspondre au mieux la taille du miroir avec la taille de l'image radiologique que peut détecter le capteur. Par exemple, si le support est suffisamment grand, on dimensionnera de préférence le miroir pour correspondre à des capteurs de dimensions moyennes : c'est-à-dire ni les plus petits ni les plus grands. De cette façon on ne perdra pas trop de précision aux extrémités de la gamme. Dans un autre exemple, si la taille du support correspond à la taille des capteurs les plus petits, ou est plus petite, on prévoira que le miroir M2 couvre entièrement la surface du support, pour offrir au praticien l'image la plus proche possible de celle fournie par le capteur. Dans ce cas il peut être préférable que le capteur soit également revêtu d'un miroir comme cela est représenté à la figure 6.

Un deuxième accessoire peut être une enveloppe 50 hygiénique, généralement à usage unique, comme représenté sur les figures 7 et 8. Une telle enveloppe est utilisée par le praticien pour améliorer les règles de propreté et d'hygiène. L'enveloppe comprend une ouverture par laquelle le capteur est introduit (Figure 7) et poussé jusqu'au fond de l'enveloppe. Après la prise du cliché radiologique, l'ensemble de prise d'image est retiré de la bouche du patient, le capteur est retiré de l'enveloppe et l'enveloppe est stérilisée, ou bien elle est jetée (enveloppe à usage unique).

L'enveloppe hygiénique est réalisée dans un matériau plastique adapté à l'usage médical et dentaire, transparent aux radiations et pouvant être transparent à la lumière visible. Mais même si elle est en effet transparente à la lumière visible, cette transparence n'est pas optimale, car sa surface est peu réfléchissante et à supposer que le capteur qu'elle enferme comprend un miroir M1 selon l'invention, sur la face active du capteur, l'image formée sur ce miroir M1 ne sera pas nette.

Selon l'invention, on peut prévoir un miroir M3 sur une face externe A" de l'enveloppe 50, dans la zone correspondant au fond de l'enveloppe.

Le capteur doit alors être introduit avec sa face active A du même côté que le miroir M3 sur la face A" de l'enveloppe. Si, comme représenté, le capteur comprend un miroir M1 sur sa face active A, les deux miroirs M1 et M2 sont alors orientés du même côté, en sorte qu'une fois placé dans la bouche du patient les deux seront orientés dans la même direction, derrière la zone anatomique à radiographier et face à la source externe de rayons X (Figure 1). C'est alors le miroir M3 qui est sur la face externe de l'ensemble de prise d'image ainsi constitué, qui va permettre au praticien de contrôler et ajuster le positionnement du capteur dans la bouche du patient, par l'observation de l'image visible qui se forme sur ce miroir.

Ce miroir M3 sera par exemple formé par une technique d'évaporation sous vide, ou bien par collage d'une pièce de miroir P comme détaillé précédemment.

Si l'enveloppe est spécifique à un type de capteur, c'est à dire ajustée précisément aux dimensions du capteur, le miroir M3 sera réalisé sur une face externe de cette enveloppe de manière à couvrir une surface équivalente à la face active du capteur, c'est-à-dire la surface interne du capot de face avant à l'exception de la bordure 24.

Si l'enveloppe est générique, pour être utilisée avec toute la gamme de dimensions des capteurs du commerce : on dimensionnera de préférence le miroir pour correspondre à des capteurs de dimensions moyennes : c'est-à-dire ni les plus petits ni les plus grands. De cette façon on ne perdra pas trop de précision aux extrémités de la gamme.

On notera que l'ensemble de prise d'image peut comprendre à la fois un accessoire support tel que l'élément 40 de la figure 6, une enveloppe hygiénique 50 et un capteur 10. Notamment, on peut avoir un élément support 40, sur lequel on va positionner, au moyen d'un adhésif prévu sur la face interne du support 41, la partie A" au droit de la face active A du capteur contenu dans une enveloppe. Après le cliché, l'ensemble est retiré de la bouche du patient, le capteur est extrait de l'enveloppe et l'élément support et/ou l'enveloppe peuvent être jetés ou stérilisés pour un nouvel usage.

Dans ce cas, selon l'invention, l'ensemble de prise d'image ainsi constitué, c'est-à-dire l'élément support 40 avec son support 41 sur lequel est disposé le capteur 10 enfermé dans une enveloppe 50, comprend au moins un miroir (M2) sur la face externe du support 41 : ce miroir qui est sur la face externe de cet ensemble de prise d'image, va permettre au praticien de contrôler et ajuster le positionnement du capteur, par observation directe de l'image qui se forme dessus.

Mais le capteur et/ou l'enveloppe de cet ensemble peuvent comprendre également un miroir respectif (M1, M3) selon l'invention.

Un autre accessoire qui peut être utilisé par le praticien, est une coque souple de confort 60 comme schématiquement illustré sur les figures 9 et 10. Ces coques de confort sont par exemple réalisées dans un matériau plastique souple à aspect de gomme lisse. Ils permettent de rendre le contact avec le capteur plus agréable (quand ce capteur est dans la bouche) et en même temps ils amortissent les effets de serrage ou de chocs éventuels entre ou contre les dents du patient.

Le praticien utilise la coque de confort comme suit : il place le capteur 10 à l'intérieur de la coque, face active A du capteur 10 plaquée contre la paroi de fond 61 de la coque 60.

Selon l'invention et comme représenté sur la figure 10, on peut prévoir un miroir M4 sur la face externe A'" de la paroi 61 de fond de coque, orientée du même côté que la face active A du capteur, derrière la zone anatomique à radiographier et face à la source externe de rayons X.

Ainsi, lorsqu'un ensemble de prise d'image constitué d'une coque de confort comprenant un miroir M4 sur la face externe, et d'un capteur 10 placé dans cette coque, est placé dans la bouche du patient, le miroir M4 va permettre au praticien de contrôler et ajuster le positionnement du capteur dans la bouche du patient, par l'observation de l'image visible qui se forme sur ce miroir. Le capteur 10 n'a pas besoin d'être revêtu d'un miroir, mais il peut l'être.

Dans le cas où le capteur est revêtu d'un miroir M1 sur sa face active A comme représenté à la figure 10, les deux miroirs M1 et M4 sont orientés du même côté, vers la zone à radiographier.

L'invention s'applique à un ensemble de prise d'image qui peut comprendre un capteur seul, ou bien un capteur en combinaison avec un ou une pluralité d'accessoires supportant ou contenant ce capteur. Selon l'invention au moins la face externe de cet ensemble est revêtu d'un miroir, tel que, lorsque l'ensemble de prise d'image (ou une partie de cet ensemble incluant le capteur) est placé dans la bouche derrière la zone à radiographier, la face réfléchissante de ce miroir est placée derrière la zone à radiographier et devant la face active A du capteur.

Si l'ensemble de prise d'image est constitué uniquement du capteur : ce miroir est le miroir M1 réalisé sur la face active du capteur.

Si l'ensemble de prise d'image est constitué d'une combinaison d'un capteur avec au moins un accessoire : au moins un miroir est réalisé sur la face externe de l'accessoire qui va se trouver juste derrière la zone à radiographier ; et le capteur et/ou le ou les autres accessoires pourront chacun être ou non revêtus d'un miroir.

Par exemple, si on envisage un ensemble de prise d'image formé d'un accessoire support 40 d'une coque de confort 60 contenant un capteur 10, cet ensemble comprend au moins un miroir M2 sur la face externe de la zone support de la coque. Il peut être avantageux de prévoir que la coque de confort 60 soit également revêtue d'un miroir tel que M4, notamment si la taille du support 41 est plus petite que la coque. Dans ce cas la face réfléchissante de ce miroir M4 sera orientée comme celle du miroir M2 et comme la face active du capteur, c'est-à-dire vers la zone à radiographier. Dans cet exemple, il n'est pas nécessaire que le boitier du capteur soit revêtu d'un miroir.

De préférence, on proposera au praticien un ensemble comportant à la fois un capteur dont le boitier comprend une face réfléchissante de miroir du côté de sa face active, un ou plusieurs éléments supports dont la face avant de support comprend une face réfléchissante de miroir, et des enveloppes ayant une partie réfléchissante. De cette manière quelle que soit la manière d'utiliser les éléments de l'ensemble, on est assuré qu'il y aura toujours une face miroir à proximité immédiate de la zone à radiographier.

En pratique, le ou les miroirs seront formés à partir d'un matériau transparent aux radiations X et compatible d'un usage médical ou dentaire, et qui offre de bonne qualité d'adhésion aux matériaux plastiques et notamment aux matériaux thermoplastiques généralement utilisés pour les coques, boitiers, élément support, et aux matériaux plastiques utilisés pour réaliser les enveloppes hygiéniques. Le ou les miroirs seront par exemple formés à partir d'un matériau métallique tel que l'aluminium, le chrome ou le nickel par exemple, en utilisant des technologies habituelles de dépôt, notamment l'évaporation sous vide, permettant des dépôts en couche mince d'épaisseur homogène, en sorte que l'intégration du ou des miroirs dans l'ensemble de prise d'image ne dégrade pas ou de manière non significative la qualité de l'image radiologique obtenue.

## Revendications

1. Ensemble de prise d'image radiologique comprenant un capteur d'image intra oral (10) comprenant une face active (A) correspondant à la surface photosensible, qui est destinée à être tournée vers une source de rayons X, **caractérisé en ce qu'**il comprend au moins un miroir (M1) qui est placé devant la face active du capteur, la face de miroir réfléchissant la lumière visible étant tournée dans le même sens que la face active du capteur.

2. Ensemble de prise d'image selon la revendication 1, dans lequel un miroir (M1) est formé sur un boîtier de protection (20) enfermant le capteur.

3. Ensemble de prise d'image selon la revendication 2, dans lequel un miroir (M1) est formé sur un capot (23) de face avant du boitier de protection, avant encapsulation et soudage du capot avec une coque (21) de face arrière du boitier.

4. Ensemble de prise d'image selon l'une quelconque des revendications 1 à 3, comprenant au moins un accessoire supportant ou contenant le capteur et une face externe de l'accessoire est placée devant la face active du capteur et tournée dans le même sens que la face active du capteur, **caractérisé en ce qu'**un miroir est formé sur ladite face externe de l'accessoire.

5. Ensemble de prise d'image selon la revendication 4, **caractérisé en ce que** l'accessoire comporte un support (41) solidaire d'une tige (42), le support présentant une face avant (A') sensiblement plane opposée à une face arrière, le capteur étant placé sur le support, face active (A) contre face arrière du support, et un miroir (M2) est formé sur la face avant (A') du support.

6. Ensemble de prise d'image selon la revendication 4, **caractérisé en ce que** l'accessoire est une enveloppe de protection hygiénique (50) enfermant le capteur (10), et l'enveloppe comprend un miroir (M3) formé sur une face externe de l'enveloppe, le capteur étant placé à l'intérieur de l'enveloppe avec sa face active (A) orientée du même coté que ladite face externe (A') de l'enveloppe et sous le miroir (M3) de l'enveloppe.

7. Ensemble de prise d'image selon la revendication 4, **caractérisé en ce que** l'accessoire est une coque souple de confort (60) à l'intérieur de laquelle est placée le capteur, face active contre une paroi de fond (61) de la coque, et un miroir (M4) est formé sur une face externe de cette paroi (61).

8. Ensemble de prise d'image selon la revendication 2, 3, 4, 5 ou 7, dans lequel le miroir est une pièce (P) découpée dans une bande de matériau ayant une face réfléchissante, intégrée par moulage en surface d'un matériau thermoplastique.

9. Ensemble de prise d'image selon l'une quelconque des revendications 1 à 7, dans lequel le miroir est formé par dépôt d'un matériau réfléchissant par évaporation sous vide.

10. Ensemble de prise d'image selon l'une quelconque des revendications 1 à 7, dans lequel le miroir est une pièce (P) découpée dans une bande de matériau ayant une face réfléchissante et une autre face adhésive.

## Patentansprüche

1. Baugruppe zur radiologischen Bildaufnahme, beinhaltend einen intraoralen Bildsensor (10), welcher eine aktive Seite (A) beinhaltet, welche der lichtempfindlichen Oberfläche entspricht, welche dazu bestimmt ist, in Richtung einer Röntgenstrahlenquelle gedreht zu werden, **dadurch gekennzeichnet, dass** sie mindestens einen Spiegel (M1) beinhaltet, der vor der aktiven Seite des Sensors platziert wird, wobei die das sichtbare Licht reflektierende Spiegelseite in dieselbe Richtung wie die aktive Seite des Sensors gedreht wird.

2. Baugruppe zur Bildaufnahme nach Anspruch 1, bei welcher ein Spiegel (M1) auf einem Schutzgehäuse (20) gebildet ist, welcher den Sensor umschließt.

3. Baugruppe zur Bildaufnahme nach Anspruch 2, bei welcher ein Spiegel (M1) auf einer Haube (23) der Vorderseite des Schutzgehäuses gebildet ist, vor Verkapseln und Verschweißen der Haube mit einer Schale (21) der Hinterseite des Gehäuses.

4. Baugruppe zur Bildaufnahme nach einem der Ansprüche 1 bis 3, beinhaltend mindestens ein Zubehörteil, welches den Sensor stützt oder diesen enthält und wobei eine äußere Seite des Zubehörteils vor der aktiven Seite des Sensors platziert ist und in dieselbe Richtung gedreht ist wie die aktive Seite des Sensors, **dadurch gekennzeichnet, dass** ein Spiegel an der äußeren Seite des Zubehörteils gebildet ist.

5. Baugruppe zur Bildaufnahme nach Anspruch 4, **dadurch gekennzeichnet, dass** das Zubehörteil eine Stütze (41) beinhaltet, die fest mit einem Stab (42) verbunden ist, wobei die Stütze eine Vorderseite (A') aufweist, welche im Wesentlichen eben ist, welche einer Hinterseite gegenüberliegt, wobei der Sensor auf der Stütze platziert ist, mit der aktiven Seite (A) gegen die Rückseite der Stütze, und ein Spiegel (M2) auf der Vorderseite (A') der Stütze gebildet ist.

6. Baugruppe zur Bildaufnahme nach Anspruch 4, **dadurch gekennzeichnet, dass** das Zubehörteil eine hygiensche Schutzhülle (50) ist, welche den Sensor (10) einschließt, und die Hülle einen Spiegel (M3) beinhaltet, welcher auf einer äußeren Seite der Hülle gebildet ist, wobei der Sensor im Innern der Hülle platziert ist, wobei seine aktive Seite (A) zur selben Seite wie die äußere Seite (A') der Hülle ausgerichtet ist und er unter dem Spiegel (M3) der Hülle platziert ist.

7. Baugruppe zur Bildaufnahme nach Anspruch 4, **dadurch gekennzeichnet, dass** das Zubehörteil eine flexible Komfortschale (60) ist, in deren Innern der Sensor platziert ist, mit der aktitven Seite gegen eine Bodenwand (61) der Schale, und ein Spiegel (M4) an einer externen Seite dieser Wand (61) gebildet ist.

8. Baugruppe zur Bildaufnahme nach Anspruch 2, 3, 4, 5 oder 7, bei welcher der Spiegel ein Teil (P) ist, welches aus einem Materialstreifen ausgeschnitten ist, welcher eine reflektierende Seite besitzt, welche durch Formen an der Oberfläche eines thermoplastischen Materials integriert ist.

9. Baugruppe zur Bildaufnahme nach einem der Ansprüche 1 bis 7, bei welcher der Spiegel durch Ablagern eines reflektierenden Materials durch Verdampfen in Vakuum gebildet wird.

10. Baugruppe zur Bildaufnahme nach einem der Ansprüche 1 bis 7, bei welcher der Spiegel ein Teil (P) ist, welches aus einem Materialstreifen ausgeschnitten ist, welcher eine reflektierende Seite und eine andere selbsklebende Seite besitzt.

## Claims

1. A radiological imaging assembly comprising an intra-oral image sensor (10) comprising an active face (A) corresponding to the photosensitive surface, which is intended to be turned toward an X-ray source, **characterized in that** it comprises at least one mirror (M1) which is placed in front of the active face of the sensor, the mirror face reflecting the visible light being turned in the same direction as the active face of the sensor.

2. The imaging assembly as claimed in claim 1, in which a mirror (M1) is formed on a protective housing (20) enclosing the sensor.

3. The imaging assembly as claimed in claim 2, in which a mirror (M1) is formed on a front face cap (23) of the protective housing, before encapsulation and welding of the cap with a rear face shell (21) of the housing.

4. The imaging assembly as claimed in any one of claims 1 to 3, comprising at least one accessory supporting or containing the sensor and an outer face of the accessory is placed in front of the active face of the sensor and turned in the same direction as the active face of the sensor, **characterized in that** a mirror is formed on said outer face of the accessory.

5. The imaging assembly as claimed in claim 4, **characterized in that** the accessory comprises a support (41) secured to a rod (42), the support having a substantially planar front face (A') opposite a rear face, the sensor being placed on the support, active face (A) against rear face of the support, and a mirror (M2) is formed on the front face (A') of the support.

6. The imaging assembly as claimed in claim 4, **characterized in that** the accessory is a hygienic protection enclosure (50) enclosing the sensor (10), and the enclosure comprises a mirror (M3) formed on an outer face of the enclosure, the sensor being placed inside the enclosure with its active face (A) oriented on the same side as said outer face (A') of the enclosure and under the mirror (M3) of the enclosure.

7. The imaging assembly as claimed in claim 4, **characterized in that** the accessory is a comfort flexible shell (60) inside which the sensor is placed, with its active face against a bottom wall (61) of the shell, and a mirror (M4) is formed on an outer face of this wall (61).

8. The imaging assembly as claimed in claim 2, 3, 4, 5 or 7, in which the mirror is a piece (P) cut from a strip of material having a reflecting face, incorporated by surface molding of a thermoplastic material.

9. The imaging assembly as claimed in any one of claims 1 to 7, in which the mirror is formed by deposition of a reflecting material by vacuum evaporation.

10. The imaging assembly as claimed in any one of claims 1 to 7, in which the mirror is a piece (P) cut from a strip of material having a reflecting face and an adhesive other face.
